Europäisches Patentamt

European Patent Office

Office européen des brevets

(19)

(11) Publication number: **0 323 663 B2**

(12)
# NEW EUROPEAN PATENT
# SPECIFICATION

(45) Date of publication of the new patent specification:
14.09.94

(51) Int. Cl.[5]: **B01J 8/02**, C07C 2/66,
C07C 15/107, C07D 301/19

(21) Application number: **88202871.5**

(22) Date of filing: **13.12.88**

(54) Exothermic reaction process in a fixed-bed catalytic reactor.

(30) Priority: **18.12.87 GB 8729555**

(43) Date of publication of application:
**12.07.89 Bulletin 89/28**

(45) Publication of the grant of the patent:
**08.04.92 Bulletin 92/15**

(45) Mention of the opposition decision:
**14.09.94 Bulletin 94/37**

(84) Designated Contracting States:
**BE DE ES FR GB IT NL**

(56) References cited:
EP-A- 0 189 683    EP-A- 0 212 689
CA-A- 929 537      DE-A- 1 470 716
DE-A- 2 755 173    GB-A- 692 176
US-A- 4 012 573    US-A- 4 100 220
US-A- 4 400 493

Chemical Engineering Progress, vol. 70, No.
1, January 1974, pp. 74-79

R. Arganbright et al. "Novel Process for
Methyl Tertiary-Butyl Ether", Final Report
Prepared for the U.S. Department Of Energy,
April 1985, DOE/CS/40454

National Petroleum Refiners Association,

1983 NPRA Annual Meeting, E. Lander et al
"The MTB Solution: Octanes, Technology
and Refinery Profitability" March 20-22, 1983,
San Francisco, California

(73) Proprietor: **SHELL INTERNATIONALE RE-
SEARCH MAATSCHAPPIJ B.V.
Carel van Bylandtlaan 30
NL-2596 HR Den Haag (NL)**

(72) Inventor: **Terlouw, Teunis
Carel van Bylandtlaan 30
NL-2596 HR The Hague (NL)**
Inventor: **van Os, George
Carel van Bylandtlaan 30
NL-2596 HR The Hague (NL)**
Inventor: **Bakker, Anke Gezina
Badhuisweg 3
NL-1031 CM Amsterdam (NL)**

## Description

The invention relates to a process for conducting exothermic reactions between two or more reactants under substantially isothermic conditions.

For many processes (that are being used) in the industry it is of the utmost importance that they be conducted under more or less isothermic conditions, as a deviation from those conditions can have a dramatic effect on process characteristics such as yield and selectivity as well as on the catalyst activity in the case of a catalyzed reaction. Hence, when such a process is based on an exothermic reaction, the control of the exotherm becomes a major issue. Although the heat generated in such a reaction may very often be dissipated by means of internal and/or external cooling, thereby providing a more or less constant reaction temperature, such cooling techniques are not always adequate or cannot always be applied. In such a situation it is sometimes possible to change the process mode to one where such coating techniques can be adequately applied, e.g. replacing a continuous process with a batchwise-operated process, or replacing a single reactor with a series of generally smaller reactors with interstage cooling and optionally a split feed operation.

In this context it is generally accepted when a process has to be conducted on a large, e.g. an industrial scale, that as a result of the presence of an exothermic reaction, the selected process mode is a compromise between the economy of the process and the methods available to control said exotherm.

The problems described hereinbefore are not necessarily restricted to the upscaling of processes but may also arise when e.g. the catalyst system in an existing process is replaced with another catalyst, e.g. a homogeneous catalyst is replaced with a heterogeneous catalyst system, as a result of which other process modes can be taken into consideration, e.g. a continuous process in a fixed-bed reactor instead of a batchwise reaction.

A typical example of a process wherein problems, as described hereinbefore, have been experienced is the preparation of alkylated aromatic compounds.

Alkylation of aromatic compounds is well-known and, as has been described in J. of Cat. 5, 81-98 (1966), may be carried out in the presence of a wide range of catalysts, such as; hydrogen fluoride (HF), aluminium chloride and silica-alumina, and under a variety of reaction conditions.

Investigations carried out by the applicant have shown that the composition of the alkylate produced via the different processes tended to vary. It was observed e.g. that the alkylate produced via the aluminosilicate-catalyzed process had a higher content of branched alkylate compared to that produced via the HF-catalyzed process. Alkylate having a high content of branched alkylate will be unacceptable for those applications where biodegradability is a primary requirement.

The difference in alkylate composition mentioned hereinbefore may well find its cause in the difference in process conditions between the HF-catalyzed process and the aluminosilicate-catalyzed process. The HF-catalyzed process is generally carried out at a temperature in the range of from 30 to 50 °C, a temperature range at which isomerization hardly occurs, while the exothermic heat generated during the alkylation reaction, is removed via external cooling. The aluminosilicate-catalyzed process on the other hand is generally carried out in a fixed-bed reactor under more or less adiabatic conditions and at temperatures > 100 °C. Under such conditions the heat generated by the exothermic alkylation reaction may result in an increase of the reactor temperature, which temperature increase, depending on the aromatic/olefin molar ratio, can be quite considerable, temperature increases of e.g. 30-40 °C being not uncommon. Furthermore, additional investigations by the applicant had shown that for a given alumino-silicate catalyst system and a given reactant composition, the alkylate produced at a higher reaction temperature was found to have a higher content of branched alkylate.

The problem underlying the present invention is developing a process for conducting the isothermic reaction between two or more reactants in a fixed-bed reactor under more or less isothermic conditions.

In EP 0189683 a process is disclosed for the preparation of alkylated aromatic compounds by effecting reaction between an olefin and an aromatic compound in a fixed-bed reactor containing a metallosilicate catalyst. In said process aromatic compounds are alkylated with olefins and the alkylate formed and the excess aromatic compound are separated via distillation, in the same reactor. In said process the heat generated by the exothermic alkylation reaction is consumed by the boil up of said aromatic compound, which is present at its boiling pressure, and hence does not result in a temperature increase in the reactor. Said process however, has a number of disadvantages which are related to the fact that the reactor is operated as a distillation unit. Firstly there is a countercurrent flow of a vapour stream in upward direction and a downward stream of a liquid phase, a situation which may give rise to backmixing, which subsequently may result in an unacceptable increase in polyalkylate content. Secondly this mode of operation requires a special catalyst arrangement within the reactor in order to provide the relatively large void volume necessary for the distillation. Finally, in order to meet the basic temperature requirements for a distillation procedure there has to be a temperature gradient over the reactor, i.e. a high temperature at the bottom of the reactor and a much lower temperature at the top. The temperature difference between top and bottom of the reactor may be quite considerable and as mentioned hereinbefore the higher temperatures will affect the isomer distribution in the alkylate produced.

It has now been found that the problems mentioned hereinbefore can be solved by conducting such exothermic reactions in a fixed-bed reactor wherein a liquid and a gas phase flow concurrently downwards, while the separation of the different compounds present or formed during the reaction is conducted outside said fixed-bed reactor.

The invention provides a process for conducting the exothermic reaction between an olefin and an aromatic compound to produce an alkylated aromatic compound, or between an olefinic compound and an organic hydroperoxide of general formula ROOH wherein R is a monovalent hydrocarbyl group to produce an oxirane compound, in a fixed-bed catalytic reactor under substantially isothermic conditions, characterized in that the reaction mixture includes at least one compound having a lower boiling point than the other compounds present, which compound is present in an amount which is at least sufficient to consume, by vaporisation thereof, the heat generated by said exothermic reaction mixture in such a manner as to provide a concurrent downflow of a liquid and a gas phase, the gas phase substantially comprising said at least one compound having the lower boiling point.

In the Journal of American Institute of Chemical Engineers, Vol. 24, No. 6, p. 1087-1104, (Nov. 1978) it is stated that the nature of the flow regime, which may occur in such a fixed-bed reactor wherein a liquid and a gas phase flow concurrently downwards, i.e. a reactor as used in the process of the present invention, is primarily determined by the flow rates of the two phases. Examples of such two-phase flow regimes which may occur include trickle flow, foaming flow and pulsing flow.

The principle of the process of the present invention can be described as follows. A reaction mixture as hereinbefore described is introduced into the more or less vertically positioned fixed-bed reactor via a gravity feed procedure. The reactor pressure is set at a value which corresponds with the boiling pressure of the reaction mixture within the reactor. The reaction commences when the reaction mixture comes into contact with the catalyst. As a result of the pressure in the reactor being the boiling pressure of said reaction mixture, the heat generated during the reaction will result in evaporation of the lowest boiling component of the reaction mixture, and not in an increase in temperature.

The effluent flowing from the bottom of the reactor, i.e. a liquid phase, which comprises amongst others the desired reaction product and a gas phase substantially comprising the vaporized compound having the lowest boiling point, are separated in one or more steps. Said compound having the lowest boiling point, as well as other unreacted compounds, which can be retrieved via said separation, may advantageously be recycled and used to make up a new feed mixture.

The process of the present invention differs from processes conducted in conventionally operated fixed-bed reactors, wherein a liquid and a gas phase flow concurrently downwards, in that with the present process the gas or vapour phase comprises a vaporized component from the liquid phase.

For a smoothly running process, it will be advantageous for the reactant feed mixture to have a temperature and pressure which do not differ too much from those in the reactor.

Although the compound having the lowest boiling point of the reaction mixture may in principle be any suitable, e.g. inert compound, it is generally preferred that said compound is one of the reactants as this may have the additional advantage in that it reduces the number of compounds in the effluent, which have to be separated. Such a reactant should be present in an amount which is at least equal to the stoichiometric amount required for the reaction product to be formed, in addition to the amount required to consume the heat generated by the exothermic reaction.

As the amount of heat generated in an exothermic reaction is amongst others related to the nature of said reaction, to the concentration wherein the reactants are present and to the degree of conversion, the amount of compound which is at least required to be present in order to consume the heat generated, will have to be calculated separately for each type of process and/or set of process conditions.

The versatility of the process of the present invention will be demonstrated hereinafter with the aid of two processes.

The first process where the present invention was successfully applied is the hereinbefore mentioned preparation of alkylated aromatic compounds wherein a reaction is effected between an olefin and an aromatic compound, which aromatic compound has a lower boiling point than said olefin, and wherein the fixed-bed contains a metallosilicate catalyst.

The process of the present invention offers the possibility of carrying out the alkylation reaction under more or less "isothermal" conditions. It has however been observed that during this process the temperature over the reactor tends to show a slight increase from top to bottom. Without wishing to be bound by any theory, it is believed that this increase in temperature is related to the change in composition of the liquid phase in the reactor. As the reaction proceeds the composition of the liquid phase gradually changes from an aromatic/olefin compound to a composition which, assuming complete olefin conversion, comprises the relatively high boiling alkylate next to considerably less low boiling aromatic compound. This change in composition will be reflected in the somewhat higher boiling temperature of the liquid phase. This temperature increase as a result of the change in composition can to a certain extent be surpressed by increasing the excess of aromatic compound in the reactant mixture. Simultaneously a larger excess of aromatic compound will also be beneficial for the selectivity of the process i.e. it will reduce the polyalkylate content of the alkylate.

The alkylation reaction may be carried out at a temperature in the range of from 70 to 300 °C, which temperature can be regulated by the pressure in the reactor. Preferably the process is carried out at a temperature in the range of from 100 to 200 °C, and more preferably at a temperature in the range of from 130 to 160 °C.

The metallosilicate catalysts which may be employed in the process of the present invention are preferably crystalline metallosilicate catalysts, which products may be of natural or synthetic origin. Aluminosilicate catalysts are especially preferred metallosilicate catalysts. Suitable such catalysts include faujasite, mordenite, zeolite X, zeolite Y, zeolite beta, ZSM-20, ZSM-12 and the like.

A preferred class of aluminosilicate catalysts are those which have been submitted to an ion exchange procedure. The ion exchange generally concerns the exchange of alkali metal ions, especially sodium, with polyvalent ions such as magnesium, calcium, zinc and especially rare earth metal ions, although exchange with ammonium ions, as an intermediate step in the preparation of such catalysts in the hydrogen form, may also be advantageous.

Especially preferred ion exchanged aluminosilicate catalysts are rare earth exchange zeolite Y catalysts having a sodium content less than 0.9 %m. The catalysts mentioned hereinbefore are known, as are methods for their preparation.

A preferred such rare earth exchanged zeolite Y catalyst is a steamed rare earth exchanged zeolite Y catalyst, the use of which has been found especially advantageous in the preparation of alkylate wherein only a very low content of branched alkylate is permissable. The steaming of said catalysts may conveniently be conducted at a temperature in the range of from 300 to 600 °C for approximately at least 30 minutes before or after extrusion of the catalyst.

The manner wherein the hereinbefore mentioned catalysts may be employed in the process of the present invention is in principle the same as in conventionally operated fixed-bed reactors and they may thus be used as supported or unsupported catalysts, and as powdered or shaped catalysts. Suitable catalyst shapes include cylinders, spheres, rings, trilobes and the like and may be prepared via known methods.

The aromatic compounds which may be alkylated by the process of the present invention are those having a boiling temperature preferably in the range of from 70 to 300 °C at the pressure in the reactor, which pressure is preferably in the range of from 0.5 to 45 bar. Examples of such aromatic compounds include mono-and polycyclic aromatic compounds as well as substituted derivatives thereof, wherein the substituents are inert under alkylation conditions. Suitable substituents include alkyl, cycloalkyl, aralkyl, alkaryl,halogen, hydroxyl, alkoxy, cyano and nitro groups. Suitable aromatic compounds include benzene, naphthalene, biphenyl, toluene, o-, m-, or p-xylene, phenol, cresol, cyclohexyl benzene, ethyl benzene, diethyl benzene and the like. Benzene, toluene, xylene and phenol are preferred aromatic compounds.

The olefins which may be used in the process of the present invention are preferably linear olefins and include both alpha- and internal olefins. Examples of suitable olefins include heptene, octene, nonene, decene, dodecene, hexadecene, octadene and the like. $C_{10}$-$C_{14}$ olefins are preferred olefins.

Although in general the reactant mixture employed in the process of the present invention will comprise mixtures of aromatic compound and olefin as such, the present invention is also considered to cover a process wherein the olefin is a blend of said olefin and the corresponding paraffin.

The second process wherein the present invention can be successfully applied is in the preparation of oxirane compounds via the catalytic epoxidation of olefins with the aid of an organic peroxide. One of the currently employed methods for conducting such a process is via a multi-reactor discipline with interstage cooling. Hence, when said process is conducted according to the present invention a considerably saving in expenditure can be achieved, both as regards the investment and the running of said process.

Such a process for the preparation of oxirane compounds according to the present invention comprises effecting a reaction between an organic hydroperoxide and an olefinic compound having a lower boiling point than the hydroperoxide and wherein the fixed bed contains a catalyst based on titanium and a solid

4

silica and/or an inorganic silicate.

The organic hydroperoxide, represented by the general formula R-O-O-H, in which R is a monovalent hydrocarbyl group, reacts with an olefinic compound to an oxirane compound and a compound R-OH.

Preferably, the group R has from 3 to 20 carbon atoms. Most preferably, it is a hydrocarbyl group, in particular a secondary or tertiary alkyl or aralkyl group, having from 3 to 10 carbon atoms. Especially preferred among these groups are the tertiary alkyl and secondary or tertiary aralkyl groups, including, e.g., tertiary butyl, tertiary pentyl, cyclopentyl, 1-phenylethyl-1, 2-phenylpropyl-2 and the various tetralinyl radicals which originate by elimination of a hydrogen atom from the aliphatic side-chain of the tetralin molecule.

Aralkyl hydroperoxide, wherein the hydroperoxy group is linked to that carbon atom of an alkyl side-chain which is attached directly to an aromatic ring, including 1-phenylethyl-1-hydroperoxide and 2-phenylpropyl-2-hydroperoxide, are often called after the corresponding hydrocarbons, e.g. ethyl benzene hydroperoxide and cumene hydroperoxide.

The organic hydroperoxide reactant used as a starting material may be in a dilute or concentrated, purified or unpurified condition.

In principle, any organic compound having at least one olefinic double bond may be reacted with an organic hydroperoxide. The compound may be acyclic, monocyclic, bicyclic or polycyclic and they may be mono-olefinic, diolefinic or polyolefinic. If there are more than one olefinic linkages, these may either be conjugated or non-conjugated. Generally preferred are olefinic compounds having from 2 to 60 carbon atoms. Although substituents, which should preferably be relatively stable, may be present, acyclic monoolefinic hydrocarbons having from 2 to 10 carbon atoms are of particular interest. Such hydrocarbons include, e.g., ethylene, propylene, isobutylene, hexene-3, octent-1 and decene-1. Butadiene may be mentioned as an example of a suitable diolefinic hydrocarbon. Substituents, if present, may, e.g., be halogen atoms or comprise atoms of oxygen, sulphur and nitrogen together with atoms of hydrogen and/or carbon. Of particular interest are olefinically unsaturated alcohols, and halogen-substituted olefinically unsaturated hydrocarbons, including, e.g., ally alcohol, crotyl alcohol and allyl chloride. Particularly preferred are alkenes having from 3 to 40 carbon atoms, which may or may not be substituted with a hydroxy or a halogen atom.

The catalyst based on titanium in combination with a solid silica and/or an inorganic silicate which may be employed in the preparation of the oxirane compounds according to the process of the present invention may conveniently be a catalyst as described in Netherlands Patent 145,233.

The catalyst may be used as a shaped or non-shaped catalyst. Suitable catalyst shapes include cylinders, spheres, rings, trilobes and the like and may be prepared via known methods.

The process for the preparation of the oxirane compounds according to the present invention will as has been mentioned hereinbefore, be conducted at the boiling pressure of the reaction mixture, which pressure will generally be in the range of from 1 to 100 bar, while the temperature in the reactor will be in the range of from 20 to 300 °C and preferably in the range of from 50 to 150 °C.

When required, the liquid phase in the reactor may also include a sufficient amount of one or more solvents and/or diluents to keep the liquid phase homogeneous. Suitable solvents which may be added include mononuclear aromatic compounds such as benzene, toluene, ethylbenzene and the like as well as halogenated aromatic compounds such as chlorobenzene, bromobenzene, orthodichlorobenzene, and also alkanes such as octane, decane and dodecane.

The process of the present invention when applied to the preparation of oxirane compounds is of special importance when it forms an integrated part of a process for the simultaneous production of an oxirane compound derived from an optionally substituted alkene, preferably having 3-4 carbon atoms and a precursor of styrene or a substituted styrene, by reaction of said alkene with an alpha-arylalkyl hydroperoxide. Suitable alkenes include propylene and alkylchloride while ethylbenzene hydroperoxide is a very suitable hydroperoxide which will upon reaction be converted into methylphenylcarbinol, which compound can subsequently be dehydrated to styrene.

The invention will be further illustrated with the following examples. Example I

Alkylation of benzene with $C_{11}$-$C_{13}$ linear internal olefins in a fixed-bed reactor operated as a trickle-bed reactor

A 4.4 l fixed-bed reactor equipped with 6 thermocouples along the reactor bed and connected with the necessary auxiliary equipment such as feed vessel, facilities for separating the alkylate and excess aromatic compound, as well as facilities for recirculating the aromatic compound, and containing a zeolite Y catalyst extrudate, wherein sodium ions had been exchanged by rare earth metal ions and which catalyst contained less than 0.6 %m sodium, was brought to a temperature of 150 °C by circulating hot benzene at a rate of 22 kg/h through the reactor having an outlet pressure of 4.8 barg. As soon as the reactor had reached a temperature of 150 °C and was operating under steady conditions the feed was switched to a vessel containing a mixture of benzene and $C_{11}$-$C_{13}$ linear internal olefins in a molar ratio of 11.25:1 and having a temperature of 151 °C and a pressure of 5.2 barg. This mixture was fed into the top of the reactor via a gravity feed procedure at a rate of 22 kg/h, which corresponds with a LHSV 5.0-5.1 kg/l cat/h while the outlet pressure of the reactor was lowered to 4.6 barg. Simultaneously with the feed switch, the effluent flow from the bottom of the reactor was switched to a separating vessel, wherein the alkylate was separated from the excess reactants. The feed stream was maintained for 3.6 h, during which period the reactor temperature was recorded via the thermocouples mentioned hereinbefore, together with the pressure at the inlet and outlet of the reactor. After 3.6 h the olefin/benzene feed mixture was replaced with benzene and benzene was recirculated until the reactor temperature had again reached 151 °C.

Analysis of the reaction product showed the olefin conversion to be 99-100 %, the dialkyl benzene content of the debenzenized reaction product to be 2.2 %m and the content of branched alkylate 9.8 %m.

The temperature and pressure data recorded during the reaction have been collected in Table 1, which shows that the temperature increase was never more than 7 °C.

Table 1

| Reaction time h | Feed temp. °C | Reactor temperature | | | | | | Pressure barg | | Remark |
|---|---|---|---|---|---|---|---|---|---|---|
| | | 17* cm | 31* cm | 56* cm | 94* cm | 143* cm | 198* cm | out | in | |
| 0 | 151 | 152 | 150 | 151 | 151 | 151 | 151 | 4.6 | 5.2 | feed in |
| 0.5 | 151 | 156 | 156 | 158 | 158 | 158 | 158 | 4.6 | 5.2 | |
| 1.0 | 151 | 155 | 155 | 157 | 158 | 158 | 158 | 4.6 | 5.2 | |
| 1.5 | 151 | 155 | 155 | 157 | 158 | 158 | 158 | 4.6 | 5.2 | |
| 2.0 | 151 | 155 | 155 | 157 | 158 | 158 | 158 | 4.6 | 5.2 | |
| 2.5 | 151 | 155 | 155 | 157 | 158 | 158 | 158 | 4.7 | 5.2 | |
| 3.0 | 151 | 155 | 155 | 158 | 158 | 158 | 158 | 4.6 | 5.2 | |
| 3.5 | 151 | 154 | 155 | 157 | 158 | 158 | 158 | 4.6 | 5.2 | |
| 3.6 | 151 | 153 | 155 | 155 | 158 | 158 | 158 | 4.6 | 5.2 | benzene in |
| 4.0 | 151 | 152 | 150 | 151 | 151 | 151 | 151 | 4.6 | 5.2 | |

* Position of thermocouples along reactor

Example II Preparation of propylene oxide via a catalytic oxidation of propene with ethylbenzene hydroperoxide

Reactor: a vertically positioned cylindrical reactor length 7.5 m, diameter 2.5 m, 60 %v of said reactor being filled with a titanium on silica catalyst.
Catalyst:
   a) Catalyst support:
$SiO_2$ granules ex Davison having a

| | |
|---|---|
| surface area : | 300 $m^2$/g. |
| pore volume : | 1.18 ml/g. |
| pore diameter : | 19 nm. |
| particle size : | 0.63-1.4 mm. |

   b) Titanium content 3.4 %m.
The catalyst is prepared following the method as described in Netherlands Patent 145,233, example VII.

When a feed mixture of 2500 kmol propene, 800 kmol ethylbenzene and 150 kmol ethylbenzene hydroperoxide, having a temperature of 100 °C, is introduced per hour into the upper part of the reactor, as described hereinbefore, and wherein the temperature is 100 °C and the inlet pressure 30 bar, this will result at the bottom of the reactor (outlet pressure 26 bar) in a gaseous effluent comprising the evaporated

6

propene and a liquid effluent which comprises 148.6 kmol propylene oxide, 148.6 kmol methyl phenyl carbinol, 232 kmol propene and 800 kmol ethylbenzene, indicating an ethylbenzene hydroperoxide conversion of 99% and a selectivity to propylene oxide of 100%.

Propylene oxide can be isolated from the liquid effluent via distillation.

## Claims

1. A process for conducting the exothermic reaction between an olefin and an aromatic compound to produce an alkylated aromatic compound, or between an olefinic compound and an organic hydroperoxide of general formula ROOH wherein R is a monovalent hydrocarbyl group to produce an oxirane compound, in a fixed-bed catalytic reactor under substantially isothermic conditions, characterized in that the reaction mixture includes at least one compound having a lower boiling point than the other compounds present, which compound is present in an amount which is at least sufficient to consume, by vaporisation thereof, the heat generated by said exothermic reaction mixture in such a manner as to provide a concurrent downflow of a liquid and a gas phase, the gas phase substantially comprising said at least one compound having the lower boiling point.

2. A process according to claim 1, characterized in that the at least one compound having the lower boiling point is a reactant.

3. A process according to claim 2, characterized in that the amount of said reactant having the lower boiling point is at least equal to the stoichiometric amount required for the reaction product to be formed, in addition to the amount required to consume the heat generated by the exothermic reaction.

4. A process according to any one of claims 1-3 wherein the reaction is between an olefin and an aromatic compound to prepare an alkylated aromatic compound, characterized in that the aromatic compound has a lower boiling point than the olefin and the fixed-bed contains a metallocilicate catalyst.

5. A process according to claim 4, wherein the reactor temperature is in the range of from 70 to 300 °C.

6. A process according to claim 4 or 5, wherein the pressure in the reactor is in the range of from 0.5 to 45 bar.

7. A process as claimed in any one of claims 4 to 6, wherein the metallosilicate catalyst is a crystalline metallosilicate catalyst.

8. A process as claimed in any one of claims 4 to 7, wherein the metallosilicate is an aluminosilicate.

9. A process as claimed in claim 8, wherein the aluminosilicate is an ion exchanged aluminosilicate.

10. A process as claimed in claim 9, wherein the ion exchanged aluminosilicate is a rare earth metal ion exchanged zeolite Y.

11. A process as claimed in claim 10, wherein the catalyst is a steamed catalyst.

12. A process as claimed in any one of claims 4 to 11, wherein the olefin is a linear olefin.

13. A process as claimed in any one of claims 4 to 12, wherein the olefin is a $C_{10}$-$C_{14}$ olefin.

14. A process as claimed in any one of claims 4 to 13, wherein the aromatic compound is selected from the group comprising benzene, toluene, xylene and phenol.

15. A process as claimed in any one of claims 5 to 14, wherein the reaction temperature is in the range of from 100 to 200 °C.

16. A process as claimed in claim 15, wherein the temperature is in the range of from 130 to 160 °C.

17. A process according to any one of claims 1-3, wherein the reaction is between an organic hydroperoxide of general formula ROOH, R being a monovalent hydrocarbyl group, and an olefinic compound to produce an oxirane compound, characterized in that the olefinic compound has a lower boiling point than the hydroperoxide and the fixed-bed contains a catalyst based on titanium and a solid silica and/or an inorganic silicate.

18. A process according to claim 17, wherein R has from 3 to 20 carbon atoms.

19. A process according to claim 17 or 18, wherein R is a secondary or tertiary alkyl or aralkyl group having from 3 to 10 carbon atoms.

20. A process according to any one of claims 17-19, wherein the hydroperoxide is selected from the group comprising ethylbenzene hydroperoxide and cumene hydroperoxide.

21. A process according to any one of claims 17-20, wherein the olefinic compound has from 2-60 carbon atoms.

22. A process according to claim 21, wherein the olefinic compound is an acyclic mono-olefinic hydrocarbon having from 2-10 carbon atoms.

23. A process according to any one of claims 17-22, wherein the pressure is in the range of from 1 to 100 bar.

24. A process according to any one of claims 17-23, which is conducted at a temperature in the range of from 20 to 300 °C.

25. A process according to claim 24, wherein the temperature is in the range of from 50 to 150 °C.

26. A process according to any one of claims 17 to 25, which forms an integrated part of a process for the simultaneous production of an oxirane compound and a precursor of styrene or of a substituted styrene by reaction of an alkene, preferably having 3-4 carbon atoms, with an alpha-arylalkyl hydroperoxide.

**Patentansprüche**

1. Verfahren zum Durchführen einer exothermen Reaktion zwischen einem Olefin und einer aromatischen Verbindung, um eine alkylierte aromatische Verbindung herzustellen, oder zwischen einer olefinischen Verbindung und einem organischen Hydroperoxid der allgemeinen Formel ROOH, in welcher R eine einwertige Kohlenwasserstoffgruppe ist, um eine Oxiran-Verbindung herzustellen, in einem Festbett-Katalysereaktor unter im wesentlichen isothermen Bedingungen,
dadurch gekennzeichnet,
daß die Reaktionsmischung mindestens eine Verbindung umfaßt, die einen niedrigeren Siedepunkt als die anderen anwesenden Verbindungen hat, wobei diese Verbindung in einer Menge vorliegt, welche zumindest ausreicht, durch Verdampfen derselben die durch die exotherme Reaktionsmischung erzeugte Wärme auf solche Weise zu verbrauchen, daß ein parallel laufender Abwärtsfluß einer flüssigen Phase und einer Gasphase erzielt wird, wobei die Gasphase im wesentlichen die mindestens eine Verbindung mit dem niedrigeren Siedepunkt umfaßt.

2. Verfahren nach Anspruch 1,
dadurch gekennzeichnet,
daß die mindestens eine Verbindung mit dem niedrigeren Siedepunkt ein Reaktionspartner ist.

3. Verfahren nach Anspruch 2,
dadurch gekennzeichnet,
daß die Menge des Reaktionspartners mit dem niedrigeren Siedepunkt mindestens gleich ist der für das zu bildende Reaktionsprodukt erforderlichen stöchiometrischen Menge zusätzlich zu der Menge, die erforderlich ist, um die durch die exotherme Reaktion erzeugte Wärme zu verbrauchen.

EP 0 323 663 B2

4. Verfahren nach irgendeinem der Ansprüche 1 bis 3,
wobei die Reaktion zwischen einem Olefin und einer aromatischen Verbindung erfolgt, um eine alkylierte aromatische Verbindung herzustellen,
dadurch gekennzeichnet,
daß die aromatische Verbindung einen niedrigeren Siedepunkt als das Olefin hat und das Festbett einen Metallsilikat-Katalysator enthält.

5. Verfahren nach Anspruch 4,
wobei die Reaktortemperatur im Bereich von 70 bis 300 °C liegt.

6. Verfahren nach Anspruch 4 oder 5,
wobei der Druck im Reaktor im Bereich von 0,5 bis 45 bar liegt.

7. Verfahren nach irgendeinem der Ansprüche 4 bis 6,
wobei der Metallsilikat-Katalysator ein kristalliner Metallsilikat-Katalysator ist.

8. Verfahren nach irgendeinem der Ansprüche 4 bis 7,
wobei das Metallsilikat ein Aluminiumsilikat ist.

9. Verfahren nach Anspruch 8,
wobei das Aluminiumsilikat ein ionenausgetauschtes Aluminiumsilikat ist.

10. Verfahren nach Anspruch 9,
wobei das ionenausgetauschte Aluminiumsilikat ein unter Verwendung eines Seltenerdmetalls ionenausgetauschter Zeolith Y ist.

11. Verfahren nach Anspruch 10,
wobei der Katalysator ein mit Dampf behandelter Katalysator ist.

12. Verfahren nach irgendeinem der Ansprüche 4 bis 11,
wobei das Olefin ein lineares Olefin ist.

13. Verfahren nach irgendeinem der Ansprüche 4 bis 12,
wobei das Olefin ein $C_{10}$-$C_{14}$-Olefin ist.

14. Verfahren nach irgendeinem der Ansprüche 4 bis 13, wobei die aromatische Verbindung ausgewählt ist aus der Gruppe, umfassend Benzol, Toluol, Xylol und Phenol.

15. Verfahren nach irgendeinem der Ansprüche 5 bis 14,
wobei die Reaktionstemperatur im Bereich von 100 bis 200 °C liegt.

16. Verfahren nach Anspruch 15,
wobei die Temperatur im Bereich von 130 bis 160 °C liegt.

17. Verfahren nach irgendeinem der Ansprüche 1 bis 3,
wobei die Reaktion zwischen einem organischen Hydroperoxid der allgemeinen Formel ROOH, wobei R eine einwertige Kohlenwasserstoffgruppe ist, und einer olefinischen Verbindung erfolgt, um eine Oxiran-Verbindung herzustellen,
dadurch gekennzeichnet,
daß die olefinische Verbindung einen niedrigeren Siedepunkt als das Hydroperoxid hat und das Festbett einen Katalysator auf Basis von Titan und ein festes Siliciumdioxid und/oder ein anorganisches Silikat enthält.

18. Verfahren nach Anspruch 17,
wobei R zwischen 3 und 20 Kohlenstoffatome umfaßt.

19. Verfahren nach Anspruch 17 oder 18,
wobei R eine sekundäre oder tertiäre Alkyl- oder Arylalkylgruppe mit 3 bis 10 Kohlenstoffatomen ist.

9

**20.** Verfahren nach irgendeinem der Ansprüche 17 bis 19,
wobei das Hydroperoxid ausgewählt ist aus der Gruppe, umfassend Ethylbenzolhydroperoxid und Cumolhydroperoxid.

**21.** Verfahren nach irgendeinem der Ansprüche 17 bis 20,
wobei die olefinische Verbindung 2 bis 60 Kohlenstoffatome umfaßt.

**22.** Verfahren nach Anspruch 21,
wobei die olefinische Verbindung ein acrylischer, mono-olefinischer Kohlenwasserstoff mit 2 bis 10 Kohlenstoffatomen ist.

**23.** Verfahren nach irgendeinem der Ansprüche 17 bis 22,
wobei der Druck im Bereich von 1 bis 100 bar liegt.

**24.** Verfahren nach irgendeinem der Ansprüche 17 bis 23,
welches bei einer Temperatur im Bereich von 20 bis 300°C ausgeführt wird.

**25.** Verfahren nach Anspruch 24,
wobei die Temperatur im Bereich von 50 bis 150°C liegt.

**26.** Verfahren nach irgendeinem der Ansprüche 17 bis 25,
welches einen wesentlichen Teil eines Verfahrens zur gleichzeitigen Herstellung einer Oxiran-Verbindung und eines Vorläufers von Styrol oder von einem substituierten Styrol bildet durch eine Reaktion eines Alkens mit vorzugsweise 3 bis 4 Kohlenstoffatomen mit einem $\alpha$-Arylalkylhydroperoxid.

## Revendications

**1.** Procédé pour mettre en oeuvre la réaction exothermique entre une oléfine et un composé aromatique afin de produire un composé aromatique aklkylé, ou entre un composé oléfinique et un hydroperoxyde de formule générale ROOH où R est un groupe hydrocarbyle monovalent afin de produire un composé oxiranne, dans un réacteur catalytique à lit fixe dans des conditions essentiellement isothermes,
caractérisé en ce que le mélange réactionnel inclut au moins un composé ayant un point d'ébullition inférieur à celui des autres composés présents, ledit composé étant présent dans une quantité qui est au moins suffisante pour absorber, par son évaporation, la chaleur engendrée par ladite réaction exothermique de façon à provoquer un écoulement concurrent vers le bas d'une phase liquide et d'une phase gazeuse, la phase gazeuse comportant essentiellement ledit, ou lesdits, composés ayant le point d'ébullition plus bas.

**2.** Procédé selon la revendication 1, caractérisé en ce que ledit composé, ou lesdits composés, ayant le point d'ébullition plus bas est, ou sont, un des réactifs.

**3.** Procédé selon la revendication 2, caractérisé en ce que la quantité dudit réactif ayant le point d'ébullition plus bas est au moins égale à la quantité stoechiométrique nécessaire pour la formation du produit de la réaction, en plus de la quantité nécessaire pour absorber la chaleur engendrée par la réaction exothermique.

**4.** Procédé selon l'une quelconque des revendications 1 à 3, dans lequel la réaction a lieu entre une oléfine et un composé aromatique afin de préparer un composé aromatique alkylé, caractérisé en ce que le composé aromatique a un point d'ébullition inférieur à celui de l'oléfine et que le lit fixe contient un catalyseur métallosilicate.

**5.** Procédé selon la revendication 4, où la température du réacteur est dans les limites de 70 à 300°C.

**6.** Procédé selon la revendication 4 ou 5, où la pression dans le réacteur se trouve dans le domaine allant de 0,5 à 45 bars.

**7.** Procédé tel que revendiqué dans l'une quelconque des revendications 4 à 6, où le catalyseur métallosilicate est un catalyseur aluminosilicate cristallin.

8.  Procédé tel que revendiqué dans l'une quelconque des revendications 4 à 7, où le métallosilicate est un aluminosilicate.

9.  Procédé tel que revendiqué dans la revendication 8, où l'aluminosilicate est un aluminosilicate avec échange d'ion.

10. Procédé tel que revendiqué dans la revendication 9, où l'aluminosilicate avec échange d'ions est une zéolithe Y avec échange d'ion de métal de terre rare.

11. Procédé selon la revendication 10, où le catalyseur est un catalyseur traité à la vapeur.

12. Procédé tel que revendiqué dans l'une quelconque des revendications 4 à 11, où l'oléfine est une oléfine linéaire.

13. Procédé selon l'une quelconque des revendications 4 à 12, où l'oléfine est une oléfine en $C_{10}$-$C_{14}$.

14. Procédé tel que revendiqué dans l'une quelconque des revendications 4 à 13, où le composé aromatique est choisi dans le groupe comportant le benzène, le toluène, le xylène et le phénol.

15. Procédé tel que revendiqué dans l'une quelconque des revendications 5 à 14, où la température de réaction est dans les limites de 100 à 200°C.

16. Procédé tel que revendiqué dans la revendication 15, où la température est dans les limites de 130 à 160°C.

17. Procédé selon l'une quelconque des revendications 1 à 3, qui comporte la préparation de composés oxirannes en effectuant une réaction entre un hydroperoxyde organique de formule générale ROOH, où R est un groupe hydrocarbyle monovalent, et un composé oléfinique ayant un point d'ébullition inférieur à celui dudit hydroperoxyde, et où le lit fixe contient un catalyseur à base de titane et d'une silice et/ou d'un silicate inorganique solides.

18. Procédé selon la revendication 17, où R a de 3 à 20 atomes de carbone.

19. Procédé selon la revendication 17 ou 18, où R est un groupe alkyle ou aralkyle secondaire ou tertiaire, ayant de 3 à 10 atomes de carbone.

20. Procédé selon l'une quelconque des revendications 17 à 19, où l'hydroperoxyde est choisi dans le groupe comportant l'hydroperoxyde d'éthylbenzène et l'hydroperoxyde de cumène.

21. Procédé selon l'une quelconque des revendications 17 à 20 où le composé oléfinique a de 2 à 60 atomes de carbone.

22. Procédé selon la revendication 21, où le composé oléfinique est un hydrocarbure acyclique mono-oléfinique ayant de 2 à 10 atomes de carbone.

23. Procédé selon l'une quelconque des revendications 17 à 22, où la pression est dans les limites de 1 à 100 bars.

24. Procédé selon l'une quelconque des revendications 17 à 23, qui est mis en oeuvre à une température dans les limites de 20 à 300°C.

25. Procédé selon la revendication 24, où la température est dans les limites de 50 à 150°C.

26. Procédé selon l'une quelconque des revendications 17 à 25, qui forme une partie intégrée d'un procédé pour la production simultanée d'un composé oxiranne et d'un précurseur du styrène, ou d'un styrène substitué, par réaction d'un alcène, ayant de préférence 3 ou 4 atomes de carbone , avec un hydroperoxyde alpha-arylalkyle.